# EUROPEAN PATENT SPECIFICATION

(11) **EP 2 658 596 B1**
(45) Date of publication and mention of the grant of the patent: **18.11.2020**
(21) Application number: 10861334.0
(22) Date of filing: 30.12.2010
(51) Int. Cl.: A61M 37/00, A61M 1/02, A61M 1/36, A61M 1/38

(54) **SYSTEM AND METHOD FOR COLLECTING PLATELETS AND ANTICIPATING PLASMA RETURN**
SYSTEM UND VERFAHREN ZUR ENTNAHME VON THROMBOZYTEN UND ANTIZIPATION VON PLASMAREINJEKTION
SYSTÈME ET PROCÉDÉ DE RECUEIL DES PLAQUETTES ET D'ANTICIPATION DE LA RÉINJECTION DU PLASMA

(43) Date of publication of application: 06.11.2013
(73) Proprietor: Haemonetics Corporation, Braintree, MA 02184 (US)
(72) Inventor: PAGES, Etienne, F-01170 Cessy (FR)
(74) Representative: Somervell, Thomas Richard
(86) International application number: PCT/US2010/062540
(87) International publication number: WO 2012/091720

(56) References cited:
- EP-A2- 1 208 856
- US-A1- 2009 217 202
- US-A1- 2010 234 788

## Description

### Technical Field

The present invention relates to systems and methods for blood processing and blood component collection, and particularly to systems and methods for collecting platelets and anticipating plasma return.

### Background Art

Apheresis is a procedure in which individual blood components can be separated and collected from whole blood temporarily withdrawn from a subject. Typically, whole blood is withdrawn through a needle inserted into a vein of the subject's arm and into a cell separator, such as a centrifugal bowl. Once the whole blood is separated into its various components, one or more of the components can be removed from the centrifugal bowl. The remaining components can be returned to the subject along with optional compensation fluid to make up for the volume of the removed component. The process of drawing and returning continues until the quantity of the desired component has been collected, at which point the process is stopped. A central feature of apheresis systems is that the processed but unwanted components are returned to the donor. Blood components separated may include, for example, a high density component such as red blood cells, an intermediate density component such as platelets or white blood cells, and a lower density component such as plasma.

Among various blood component products obtainable through apheresis, the demand for plasma reduced platelet products is rapidly growing. This is particularly because, with the improvement in cancer therapy, there is a need to administer more and more platelets to patients with lowered hemopoietic function, but the same patients may not need to be transfused with the plasma used to suspend platelets. Platelets are fragments of a large cell located in the marrow called a megakaryocyte and primarily contribute to hemostasis by performing aggregation function. Platelets also have a role in tissue healing. Normal platelet counts in adults are 150,000-400,000/mm³. Platelet counts under 20,000/mm³ can cause various troubles such as spontaneous bleeding.

Platelets have a short half-life of 4-6 days and the number of donors is limited. Therefore, in producing plasma reduced platelet products, it is important to harvest platelets from the whole blood supplied by a donor at a maximum yield and in a required amount. Further, it is known that the contamination of plasma reduced platelet product by white blood cells can lead to serious medial complications, such as GVH reactions. Therefore, it is also very important to keep the level of contamination by white blood cells as low as possible, while efficiently collecting platelets. To this end, various techniques have been developed. For example, using "surge" technology, after whole blood is collected and concentrically separated within a centrifuge into higher density, intermediate density and lower density components and plasma is harvested (so-called "draw" step), plasma is supplied through the centrifuge at a surge flow rate (e.g., a flow rate that increases with time). By performing the surge, platelets can be preferentially displaced from the intermediate density components (which exist as a buffy coat mainly comprising a mixture of platelets and white blood cells), and plasma reduced platelet products can be produced at an increased yield.

Instead of using surge technology, the platelet layer can also be extracted from the centrifuge by means of a layer "push" in which anticoagulated whole blood is introduced into the bowl until the platelet layer is pushed out, or by using a combination of surge and push methodologies. After harvesting a desired component or components, the residual blood components mostly comprising red blood cells and citrated plasma are returned to the donor (so-called "return" step).

As mentioned above, in many blood apheresis procedures and applications, unwanted components (e.g., the components that are not collected) are returned to the donor. In addition to contamination concerns (e.g., contamination, particulates, etc. being returned to the donor), the comfort of the subject must be taken into consideration. For example, returning citrated plasma to the subject too quickly or returning too much citrated plasma to the subject at one time can cause significant discomfort to the patient. Additionally, care must be taken to limit the volume of fluid outside of the body (e.g., extra-corporeal volume) and/or the reduction of fluid in the body (e.g., intra-vascular deficit).

US 2010/234788 discloses a system for the re-anticoagulation of platelet rich plasma (PRP) that includes priming the system such that a volume of the anticoagulant is transferred to a PRP container. The anticoagulant in the PRP container may then be transferred to a red blood cell container, and a volume of PRP collected in the PRP container. At various points in the process a portion of the anticoagulant within the red blood cell container is transferred to the PRP container. To help dilute the anticoagulated whole blood entering the separation bowl, the plasma collected within a plasma container may be recirculated into the bowl. In a return step during which the rotation of the bowl is stopped the remaining blood components within the bowl are returned to the donor by reversing a draw pump. During this return step, a second pump may be used to mix plasma within the plasma container with the returning components from the bowl to dilute the returning red blood cell component. A tube fluidly connects the PRP container and the bowl, so that platelets/PRP may be drawn from the container and reintroduced into the bowl for further processing.

EP 1 208 856 A2 discloses a platelet collecting apparatus that includes a centrifugal separator, a first line for allowing blood to enter the separator, a second line for allowing blood to emanate from the separator, and a plasma collection bag connected to the first and second lines. In use, anticoagulated whole blood is introduced into the separator, where it is separated into a plasma layer, a buffy coat layer, and an erythrocyte layer. When the separator is full, collection of blood is stopped, and a path opened to collect plasma within the plasma collection bag. The centrifugal separator is actuated to circulate plasma from within the collection bag to the separator. Platelets are then collected by causing the plasma in the bag to flow into the separator which expels the platelets into the platelet collection bag. After subsequently collecting a prescribed amount of the buffy coat in a bag the components remaining in the separator are returned by reversing the rotation of the pump. A buffy coat return step may be included in which the pump draws buffy coat from the buffy coat bag and supplies it to the separator.

US 2009/217202 A1 discloses another apparatus for collecting platelets from blood.

### Summary of the Invention

According to the present invention there is provided a system for collecting plasma reduced platelets and anticipating plasma return as set forth in claim 1. Insofar as the term invention or embodiment is used in the following, or features are presented as being optional, this should be interpreted in such a way that the only protection sought is that of the invention claimed.

In accordance with one embodiment of the invention, a method for collecting plasma reduced platelets and anticipating plasma return is presented. Whole blood is first drawn from the donor, anticoagulated, and introduced into a separation chamber. The separation chamber separates the anticoagulated whole blood into a number of blood components. The method then transfers plasma separated from the anticoagulated whole blood to a plasma container, and returns a first volume of the plasma from the plasma container back to the donor. The method then repeats the drawing, anticoagulating, introducing, and transferring steps to fill the separation device with additional anticoagulated whole blood.

Once the anticoagulated whole blood is introduced into the separation chamber (e.g., to fill the chamber), the method extracts platelet rich plasma ("PRP") from the separation chamber (e.g., using a surge elutriation method and/or surging with plasma) and into a PRP container. The method may then return the blood components remaining within the separation chamber to the donor and repeat the drawing, anticoagulating, and introducing steps to, once again, partially fill the separation device with anticoagulated whole blood. Once the separation device is partially filled, the method may then reintroduce PRP from the PRP container into the separation chamber, transfer plasma from the separation chamber to the plasma container, and reprocess the reintroduced PRP to create an enlarged layer of platelets within the separation device.

In accordance with some embodiments, the method may anticipate plasma return and return plasma within the plasma container to the donor while reintroducing the PRP into the separation chamber and/or reprocessing the PRP. The method may then, once again repeat the drawing, anticoagulating, and introducing steps to fill the separation chamber, remove the enlarged layer of platelets within the separation device using a surge elutriation method such that the platelets are transferred to a platelets container. The method may then return the blood components remaining within the separation chamber to the donor.

In accordance with related embodiments, the method may also return plasma from the plasma container to the donor during dead times (times during which current apheresis procedures neither draw nor return blood or blood components to the donor). Additionally, the method may calculate an extra-corporeal volume and/or an intra-vascular deficit, and the first volume of plasma returned to the donor may be based, at least in part, upon the calculated extra-corporeal volume or intra-vascular deficit.

In accordance with another embodiment of the present invention, a system for collecting plasma reduced platelets and anticipating plasma return includes a venous access device, a blood component separation device, a first return line, a recirculation line, and a second return line. The venous access device is configured to draw a first volume of whole blood from a subject and return blood components to the subject using a first pump. The blood component separation device (e.g., a centrifuge bowl) separates the drawn blood into a first blood component (e.g., plasma) and a second blood component (e.g., platelets), and is configured to send the first blood component to a first blood component bag and the second blood component to a second blood component bag.

The first return line fluidly connects the venous-access device and the blood component separation device and is configured to return blood components remaining in the separation device to the subject. The recirculation line connects the first blood component container and the separation device. The second return line fluidly connects the first blood component container and the first return line, and is configured to return the first blood component within the first blood container to the subject. The first blood component within the first blood component container may also be reintroduced into the separation chamber through the recirculation line and a recirculation pump.

The first pump may be used to return the first blood component within the first blood component container, and the blood components remaining in the separation device to the subject. The separation device may also separate the whole blood into a third blood component (e.g., red blood cells) in addition to the first and second blood components, and the blood components remaining in the separation device may include the third blood component.

In some embodiments, the second blood component may be removed from the separation device using a surge elutriation method. The surge elutriation method includes reintroducing the first blood component into the blood component separation device through the recirculation line at an increasing rate until the second blood component is removed from the blood component separation device. The second blood component may be platelets and may be reintroduced into the blood component separation device after a predetermined amount of platelets is collected within the second blood component container so as to extract a plasma reduced platelet product from the separation device.

The system may also include an anticoagulant line and a reintroduction line. The anticoagulant line may be connected to an anticoagulant source, and may introduce anticoagulant into the drawn blood. The reintroduction line may fluidly connect the second blood component bag and the blood component separation device. The second blood component within the second blood component bag may be reintroduced into the blood component separation device when a second volume of whole blood is withdrawn from the subject to create an enlarged layer of the second blood component within the blood component separation device. The enlarged layer of second blood component may be removed from the blood component separation device using a surge elutriation method.

The system may return the first blood component to the subject as the second blood component is reintroduced into the blood component separation device and/or during dead times. The system may also include a controller that calculates at least one of an extra-corporeal volume and an intra-vascular deficit. The system may then return a volume of first blood component via the second return line based, at least in part, upon the calculated extra-corporeal volume or intra-vascular deficit.

In accordance with still further embodiments of the present invention, a system for collecting plasma reduced platelets may include (1) means for drawing a first volume of whole blood from a subject and returning blood components to the subject, (2) a blood component separation means for separating the drawn blood into a first blood component and a second blood component, (3) a first return means fluidly connecting the means for drawing a first volume of whole blood and the blood component separation means and for returning blood components remaining in the separation means to the subject, (4) a recirculation means connecting the second blood component container and the separation means, and (5) a second return means fluidly connecting the first blood component container and the first return means. The blood component separation means may be configured to send the first blood component to a first blood component bag and the second blood component to a second blood component bag. The recirculation means may reintroduce the second blood component within the second blood component container into the separation means. The second return means may be configured to return the first blood component within the first blood container to the subject.

The means for drawing whole blood may include a first pump that is configured to return the first blood component within the first blood component container to the subject. The first pump may also return the blood components remaining in the separation means to the subject.

In accordance with additional embodiments, the system may also include a controller for controlling the flow of fluids through the system. The controller may repeatedly draw whole blood from the donor into the separation means, extract first and second blood components from the separation means, return first blood components to the subject using the second return means, and return remaining components in the separation means back to the subject using the first return means. Additionally, after a predetermined volume of second blood component has been sequestered in the second blood component container, the second blood component from the second blood component container may be reintroduced into the separation means. The controller may also calculate an extra-corporeal volume and/or an intra-vascular deficit, and the amount of plasma returned using the second return means may be based (at least in part) on the extra-corporeal volume and/or an intra-vascular deficit.

Moreover, the system may also include means for introducing anticoagulant into the drawn whole blood, and a reintroduction means fluidly connecting the second blood component bag and the blood component separation means. The second blood component within the second blood component bag may be reintroduced into the blood component separation means when a second volume of whole blood is withdrawn from the subject to create an enlarged layer of the second blood component within the blood component separation means. The enlarged layer of second blood component may be removed from the blood component separation means using a surge elutriation method. Additionally or alternatively, the second blood component may be platelets and may be reintroduced into the blood component separation means after a predetermined amount of platelets is collected within the second blood component container so as to extract a plasma reduced platelet product from the separation means.

In accordance with additional embodiments, a method for collecting plasma reduced platelets and anticipating plasma return is presented. Whole blood is first drawn from a source, anticoagulated, and introduced into a separation chamber. The separation chamber separates the anticoagulated whole blood into a number of blood components. The method then transfers plasma separated from the anticoagulated whole blood to a plasma container, and returns a first volume of the plasma from the plasma container to the source. The method then repeats the drawing, anticoagulating, introducing, and transferring steps to fill the separation device with additional anticoagulated whole blood.

Once the anticoagulated whole blood is introduced into the separation chamber (e.g., to fill the chamber), the method extracts platelet rich plasma ("PRP") from the separation chamber (e.g., using a surge elutriation method and/or surging with plasma) and into a PRP container. The method may then return the blood components remaining within the separation chamber to the source, and repeat the drawing, anticoagulating, and introducing steps to, once again, partially fill the separation device with anticoagulated whole blood. Once the separation device is partially filled, the method may then reintroduce PRP from the PRP container into the separation chamber, transfer plasma from the separation chamber to the plasma container, and reprocess the reintroduced PRP to create an enlarged layer of platelets within the separation device.

In accordance with some embodiments, the method may anticipate plasma return and return plasma within the plasma container to the source while reintroducing the PRP into the separation chamber and/or reprocessing the PRP. The method may then, once again repeat the drawing, anticoagulating, and introducing steps to fill the separation chamber, remove the enlarged layer of platelets within the separation device using a surge elutriation method such that the platelets are transferred to a platelets container. The method may then return the blood components remaining within the separation chamber to the source.

In accordance with related embodiments, the method may also return plasma from the plasma container to the source during dead times. Additionally, the method may calculate an extra-corporeal volume and/or an intra-vascular deficit, and the first volume of plasma returned to the source may be based, at least in part, upon the calculated extra-corporeal volume or intra-vascular deficit.

In still further embodiments, a method for collecting plasma reduced platelets may include drawing whole blood from a source, introducing anticoagulant into the whole blood drawn from the source, and introducing the anticoagulated whole blood into a separation chamber. The separation chamber, in turn, may separate the anticoagulated whole blood into a number of blood components. The method may then transfer plasma separated from the anticoagulated whole blood to a plasma container, return a first volume of the plasma from the plasma container back to the source, and repeat the above steps. Additionally, the method may also extract platelet rich plasma from the separation chamber into a platelet rich plasma container, and remove the remaining blood components from the separation chamber.

### Brief Description of the Drawings

The foregoing features of the invention will be more readily understood by reference to the following detailed description, taken with reference to the accompanying drawings, in which:
Fig. 1 is a schematic diagram of an apheresis machine, in accordance with one embodiment of the invention;
Fig. 2 is a schematic diagram of a disposable system for use with the machine of Fig. 1, in accordance with one embodiment of the invention;
Fig. 3 is a side view of a centrifuge bowl for use with the machine of Fig. 1, in accordance with one embodiment of the invention; and
Fig. 4 is a flow chart depicting a method for collecting plasma reduced platelets from a donor and anticipating plasma return, in accordance with one embodiment of the invention.

### Detailed Description of Specific Embodiments

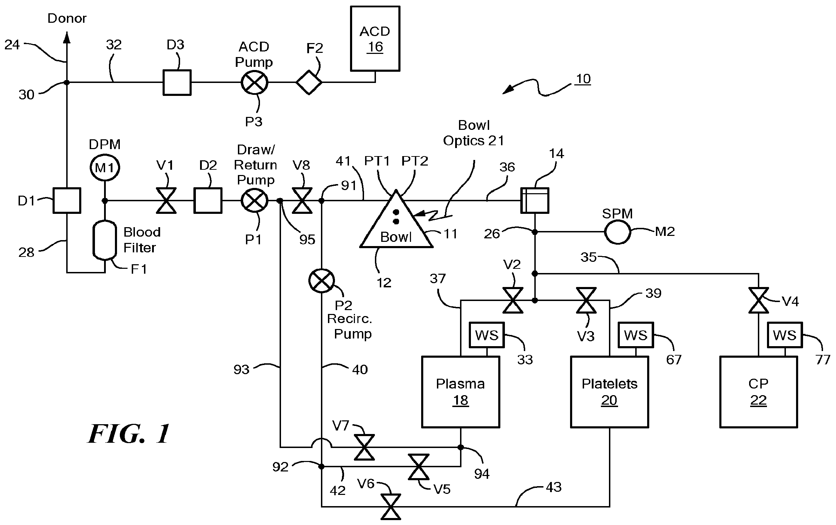

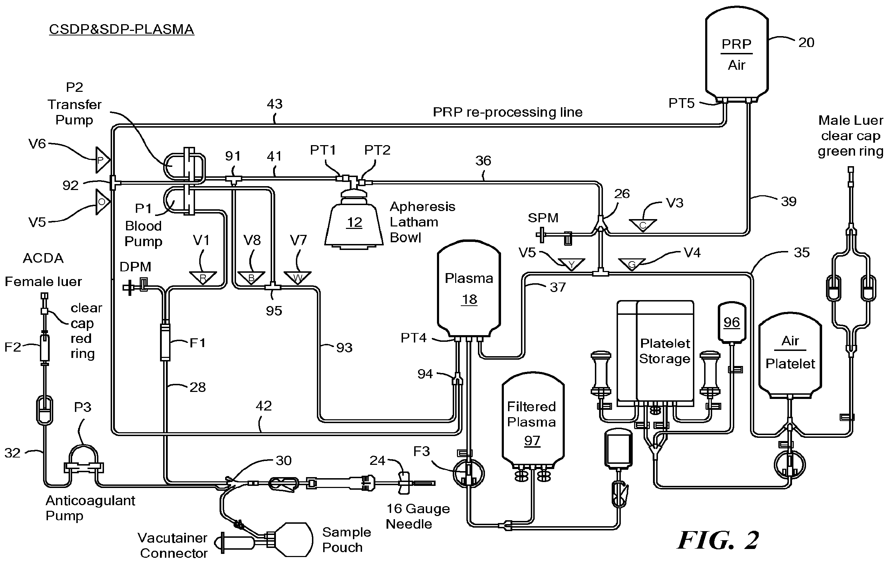

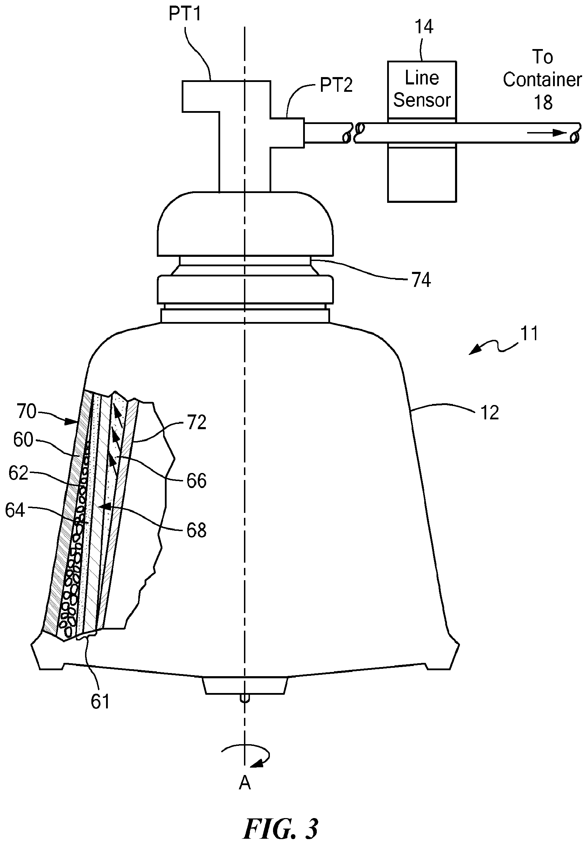

Referring to Figs. 1 and 2, an apheresis apparatus 10 uses a blood component separation device, such as a standard Latham type centrifuge 11 for separating anticoagulated whole blood into its constituent components, as described in US Patent No. 3,145,713, which is hereby incorporated by reference. Other types of separation chambers and devices may be used, such as, without limitation, an integral blow-molded centrifuge bowl, as described in U.S. Pat. Nos. 4,983,156 and 4,943,273. The centrifuge 11 includes a rotating bowl 12 and stationary input and output ports PT1 and PT2 that are typically closely coupled to the bowl interior by a rotary seal 74 (see Fig. 3). The input port PT1 of the centrifuge 11 is in fluid communication with a venous access device 24 (e.g., a phlebotomy needle) via a blood micro-aggregate filter F1, a tube 28 and a Y-connector 30 when a valve V1 is open. The venous access device 24 may be replaced with a whole blood bag (not shown) in case the whole blood is to be first pooled and then supplied. The tube 28 has compatibility with blood, as is all the tubing in the apparatus 10. The outlet port PT2 of the centrifuge 11 is selectively coupled by a tube 36, a valve V2 and a tube 37 with a first container 18 labeled plasma suspended from a weight scale 33. A second container 20 labeled platelets is selectively coupled via the tube 36, a valve V3 and a tube 39 to the outlet port PT2. Additionally, a third container 22 labeled platelet storage is selectively coupled via the tube 36, a valve V4 and a tube 35 to the outlet port PT2. Both second container 20 and third container 22 may also be suspended by weight scales 67 and 77, respectively. As discussed in greater detail below, some embodiments may also include a tube 93 fluidly connected to the plasma container 18 at one end (via connector 94) and tube 41 at the other end (e.g. via connector 95). The tube 93 may be used to return citrated plasma within the plasma container 18 to the donor during dead times and/or while processing the whole blood within the centrifuge 11.

A bag or container 16 for storing an anticoagulant is in fluid communication with the venous access device/phlebotomy needle 24 via a bacteria filter F2, a tube 32 and the Y-connector 30. The bacteria filter F2 prevents any bacteria in the anticoagulant (ACD) container 16 from entering the system. Containers 16, 18, 20, and 22 are preferably plastic bags made of a blood compatible material. Peristaltic pumps P1, P2 and P3, together with the valves V1, V2, V3, and V4 control the direction and duration of flow through the apparatus 10 in response to signals generated by a line sensor 14, a donor pressure monitor (DPM) M1, a system pressure monitor (SPM) M2 and air detectors D1, D2 and D3. The air detectors D1, D2 and D3 detect the absence or presence of fluid. The pressure monitors M1 and M2 monitor pressure levels within the apparatus 10. The line sensor 14 is an optical sensor and detects the presence of blood components passing through the line sensor 14 from the output port PT2.

In initial operation, the pumps P1 and P3 are energized to prime the tube 28 of the apparatus 10 with the anticoagulant from the container 16. The anticoagulant passes through the filter F2 and Y-connector 30 before reaching the air detector D1. The air detector D1 senses the presence of the anticoagulant at D1 and terminates the anticoagulant priming operation. During the priming operation, the valve V2 is open and sterile air displaced from bowl 12 by the anticoagulant enters the top port PT3 of the plasma container 18. The venous access device 24 is then inserted into the donor and the draw step is ready to be commenced.

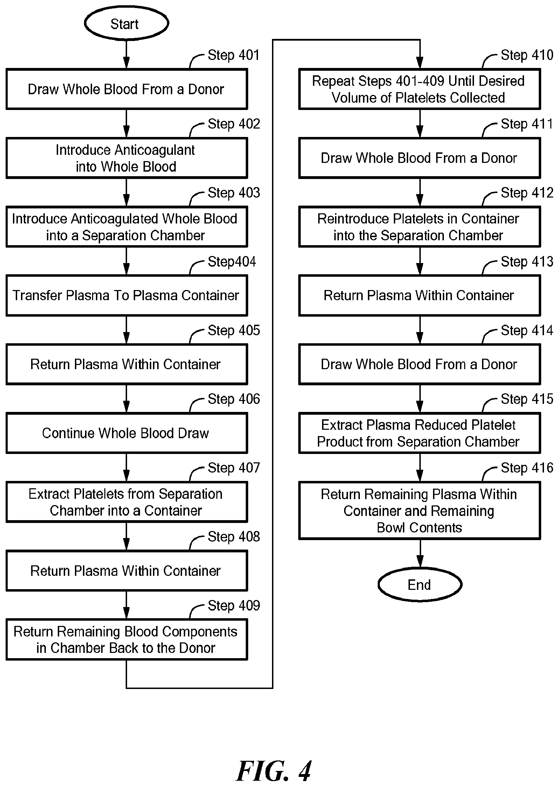

Fig. 4 is a flowchart depicting a method for collecting blood components (e.g., platelets) from a subject and anticipating return of citrated plasma to the subject, in accordance with one embodiment of the invention. During draw step 401, whole blood is drawn from the subject, typically at a rate of about 80 ml/min. and mixed with the anticoagulant using the pumps P1 and P3 (Step 402) (referring back to Figs. 1-2). The pump P3 mixes the anticoagulant from the container 16 with the whole blood drawn from the subject or a bag in which it is pooled. The valve V1 is open, allowing the anticoagulated whole blood to pass through the tube 28 and blood filter F1 before being pumped into the separation device 12 through the inlet port PT1.

The whole blood is introduced into the bottom of the separation device 12 through a feed tube (not shown), step 403 of Fig. 4. The ratio of the anticoagulant to whole blood is typically about 1:10. The operation of each of the pumps and valves in the apheresis apparatus 10 can be performed in accordance with desired protocols under the control of a controller (not shown), which may be, for example, a microprocessor.

Referring to FIG. 3, the centrifuge 11 has the fixed inlet port PT1 and the fixed outlet port PT2. The rotary seal 74 fluidly couples the stationary inlet port PT1 to the lower interior portion of the bowl 12, and the outlet port PT2 to an upper portion of the bowl interior for collecting separated fractions. A core 72 occupies a volume coaxial with the interior of bowl 12 and provides a separation region between the wall of the core 72 and the outer bowl wall 70.

As the bowl 12 is rotated, centrifugal forces separate the anticoagulated whole blood admitted into the bottom of the bowl into red blood cells (RBC), white blood cells (WBC), platelets and plasma. The number of rotations of the bowl 12 can be selected, for example, within a range of 4,000 to 6,000 rpm, and is typically 4,800 rpm. The blood is separated into different fractions in accordance with the component densities. The higher density component, i.e., RBC 60, is forced to the outer wall 70 of the bowl 12 while the lower density plasma 66 lies nearer the core 72. A buffy coat 61 is formed between the plasma 66 and the RBC 60. The buffy coat 61 is made up of an inner layer of platelets 64, a transitional layer 68 of platelets and WBC, and an outer layer of WBC 62. The plasma 66 is the component closest to the outlet port from the separation region and is the first fluid component displaced from the bowl 12 via the outlet port PT2 as additional anticoagulated whole blood enters the bowl 12 through the inlet port PT1. As the plasma 66 is displaced from the bowl 12, it is transferred to the plasma container 18 through lines 36 and 37 (Step 404 of Fig. 4).

Returning to Fig. 1, the displaced plasma passes through the line sensor 14, the tube 36, a 3-way T-connector 26, and the valve V2 (in the open position) and enters the first container 18. The plasma entering the first container 18 is drawn from the container 18 by the pump P2 via tube 42, valve V5 (in the open position), Y-connector 92 and tube 40 from the lower port PT4 and is recirculated into the bowl 12 through the inlet port PT1 via Y-connector 91 and line 41. The recirculated plasma dilutes the anticoagulated whole blood entering the bowl 12 and allows the blood components to separate more readily. An optical sensor 21 is applied to a shoulder portion of the bowl 12 for monitoring each layer of the blood components as they gradually and coaxially advance toward the core 72 from the outer wall 70 of the bowl 12. The optical sensor 21 may be mounted in a position at which it can detect the buffy coat reaching a particular radius, and the steps of drawing the whole blood from the donor 401 and introducing the whole blood into the bowl 402 may be terminated in response to the detection.

The amount of whole blood processed by the bowl 12 may be varied in response to at least one characteristic associated with the whole blood, such as the hematocrit value, the number of platelets, the total amount of blood or the like of the whole blood, as described in copending U.S. patent application 09/392,880, filed September 9, 1999, entitled Apheresis Apparatus and Method for Producing Blood Products. This variable control can be implemented under the control of a microcomputer, as aforementioned. Alternatively, each of them can be implemented manually.

As mentioned above, some embodiments of the present invention return plasma within the plasma container (e.g., citrated plasma) at various intervals during the apheresis process in order to anticipate plasma return and avoid returning a large volume at the end of the apheresis process (e.g., to improve patient comfort). To that end, the system 10 may pause the draw step (Step 401), and return some or all of the plasma contained within plasma container 18 to the donor. In particular, once the draw step (Step 401) is paused, the apheresis system 10 may reverse the direction of the draw/return pump P1, open valve V6, draw plasma from the plasma container 18 through outlet port PT4, and return the plasma to the patient/donor via lines 93 and 28.

The volume of plasma returned to the patient/donor during this step may depend upon a number of factors including, but not limited to the extra-corporeal volume ("ECV") and/or intra-vascular deficit ("IVD"). For example, the controller may calculate the ECV or IVD based upon data from previous cycles (e.g., previous cycles in a given apheresis procedure or cycles from a prior procedure), and determine if the ECV/IVD exceeds a predetermined percentage of estimated total blood volume ("TBV") (e.g., as estimated from donor characteristics such as gender, size, and weight). If the system 10 (e.g., the controller/microprocessor) determines that the ECV/IVD exceeds the predetermined percentage of TBV, then the system 10 may pause the draw (e.g., Step 401) and return plasma as discussed above. Additionally or alternatively, the system 10 may initiate the plasma return (Step 405) if the volume of plasma separated from the current cycle is equal to the volume of whole blood still to be drawn to complete the bowl filling. In order to preserve and/or improve blood separation during the plasma return step (Step 405), the centrifuge 11 may continue to spin and plasma may be circulated through the bowl 12 (e.g., using pump P2 and line 42).

By pausing the draw step (Step 401) and initiating the anticipated plasma return step (Step 405), the system 10 is able to greatly reduce the ECV and IVD. Additionally, the anticipated plasma return process also ensures that the return of citrated plasma to the patient/donor occurs over multiple steps with a significant time interval (e.g., several minutes) between. As discussed above, this helps improve patient comfort and reduces the risks associated with returning citrated plasma to the patient/donor.

Once the plasma return step is complete, the system may continue to draw whole blood from the patient/donor (e.g., in order to complete bowl filling) (Step 406) and extract platelets from the separation chamber 11/bowl 12. The platelets are extracted from the bowl 12 into a container, step 407 of Fig. 4. When extracting the platelets from the bowl 12, various methodologies may be employed, including, without limitation, dwell, surge, and/or push methodologies. For illustrative purposes, platelet extraction based on a dwell and surge technique will now be described in detail.

After the whole blood has been introduced into the centrifuge 11, step 406 of Fig.4, the valve V1 is closed and the pump P1 is stopped so that blood is no longer drawn from the donor, and dwell is commenced. During the dwell, the pump P2 recirculates plasma 66 through the bowl 12 at a moderate rate (for example, about 100 ml/min. in Fig. 4) for about 20 to 30 seconds. At this flow rate, the buffy coat 61 is diluted by the plasma and widens but the platelets do not leave the bowl 12. The dilution of the buffy coat allows the heavier white blood cells to sediment to the outer side of the buffy coat, resulting in a better separation between the lighter platelets layer 64 and the heavier white blood cells layer 62. As a result, the transitional layer 68 is reduced. The dwell period also allows the flow patterns in the bowl 12 to stabilize and allows more time for microbubbles to leave the bowl 12 and be purged.

After dwell, the surge step is commenced. In the surge, the speed of the pump P2 is increased in 5-10 ml/min. increments to recirculate plasma until reaching a platelet surge velocity of about 200-250 ml/min. The platelet surge velocity is the velocity at which platelets can leave the bowl 12 but not red blood cells or white blood cells. The plasma exiting the bowl becomes cloudy with platelets and this cloudiness is detected by the line sensor 14. The line sensor 14 consists of an LED which emits light through blood components leaving the bowl 12 and a photo detector which receives the light after it passes through the components. The amount of light received by the photo detector is correlated to the density of the fluid passing through the line.

When platelets first start leaving the bowl 12, the line sensor output starts to decrease. The valve V3 is opened and the valve V2 is closed and the platelets are collected in container 20. Once the majority of the platelets are removed from the bowl 12, the fluid exiting the bowl becomes less cloudy. This lessening of cloudiness is detected by the line sensor 14, whereupon valve V3 is closed.

It is important to note that during any "dead" times (e.g., time during which whole blood is not being drawn from the patient/donor, any time during which the centrifuge 11 is stopped, etc), the system 10 may return plasma contained within the plasma bag 18 to the patient/donor. For example, while the system 10 removes/extracts the platelets from the centrifuge bowl 12 and/or during the dwell step described above, the system 10 may also return plasma contained within the container 18 to the patient/donor in a manner similar to that described above (e.g., by reversing the pump P1 and drawing the plasma through line 93.

If the plasma return occurs during the surge process (e.g., the recirculation of plasma through the bowl 12), both the draw/return pump P1 and the recirculation pump P2 may draw plasma from the plasma container 18 and both valves V5 and V7 may be open. The draw/return pump P1 may draw the plasma through tube 93 and the recirculation pump P2 may draw plasma through tube 42. To prevent the separate plasma flows from mixing and interfering with one another, valve V8 (Fig. 2), located between connectors 91 and 95 may be closed.

After the platelets have been collected, return step 409 (see Fig. 4) is initiated. During return step 409, the rotation of the bowl 12 is stopped and the remaining blood components in the bowl 12 are returned to the donor by reversal of rotation of the pump P1 via the venous access device 24 with the valve V1 open. The valve V2 is also opened to allow air to enter the centrifuge bowl during the return. The plasma from the container 18 dilutes the remaining blood components in the bowl 12. Namely, the pump P2 mixes the plasma with the returning components in the bowl 12 with the valve V2 open, diluting the returning red blood cells component with plasma to speed up the return time. When the remaining blood components in the bowl have been returned to the donor, the return step 409 is terminated.

Referring to Fig. 4, the steps of drawing whole blood from the donor (Step 401), introducing anticoagulant into the whole blood (Step 402), introducing the whole blood into a separation chamber (Step 403), transferring plasma to the plasma container 18 (Step 404), returning plasma to the patient/donor (Step 405), continuing whole blood draw (Step 406), extracting platelets from the separation chamber (Step 407), returning plasma (Step 408), and returning the remaining components back to the donor (Step 409), are repeated until a desired volume of platelets is sequestered in the container 20, step 410. Typically, steps 401-409 are repeated two to four times, with about 450-500 ml of whole blood processed per cycle. The sequestered platelet concentration is typically about 1.5 x10⁶/µL.

The system may then re-process the platelets within container 20 by reintroducing the platelets (platelet rich plasma) in container 20 into the bowl 12, step 412 of Fig. 4. Reintroducing the platelets forms a layer of platelets that is several times larger than that obtained by processing only one cycle of whole anticoagulated blood. For example, in some embodiments, the platelet layer volume is approximately equal to the average volume of one cycle multiplied by the number of platelet sequestering cycles plus one. The platelets are drawn from port PT5 of container 20 by pump P2 via tube 43, valve V6 (in the open position), Y-connector 92, and tube 40, and input into bowl 12 through the inlet port PT1 via Y-connector 91 and line 41. To minimize contact between the platelets and bowl 12, the bowl 12 may be partly filled with anticoagulated whole blood drawn from the donor 401 prior to re-introduction of the platelets, step 411 of Fig. 4. The whole blood forms a cell bed at the periphery of the bowl 12 that serves as a buffer between the periphery of the bowl and the platelets, reducing platelet clumping. Additionally or alternatively, whole anticoagulated blood may be added to the separation chamber during platelet reintroduction so as to bring platelet layer towards the elutriation radius, or after platelet reintroduction for perfecting platelet separation and standardizing conditions of initiating platelet extraction.

It is worth noting that, if the platelets being reintroduced into the bowl 12 and reprocessed are in the form of platelet rich plasma ("PRP")(platelets suspended within plasma), the centrifugal forces within the bowl 12 will cause the PRP to abandon the suspended platelets, thereby freeing the plasma. This freed plasma will be displaced from the bowl 12 and sent to the plasma container 18 as additional fluid (e.g., additional PRP, plasma, anticoagulated whole blood, etc.) enters the bowl 12.

As the platelets are reprocessed to form the plasma reduced platelet product (discussed in greater detail below), the system 10 may, once again, return plasma within the container 18 to the patient/donor (Step 413). For example, as the platelets from container 20 are being reintroduced into the bowl 12, the system 10 may open valve V7 and use the draw/return pump P1 to draw plasma from port PT4 of container 18 through line 93, and return the plasma to the patient/donor through line 28 and the venous access device 24. The system 10 may then draw whole blood from the patient/donor and fill the bowl 12 with anticoagulated whole blood, step 414.

Using, for example, surge or push methodologies, the plasma reduced platelet concentrate is then extracted from the layer of platelets that now reside in bowl 12, step 415 of Fig. 4. The plasma reduced platelet product is sequestered in container 22 via line sensor 14, tube 36, 3-way T-connector 26 and valve V4 (in the open position). Platelet product concentration is typically in the range of 2.6x10⁶/µL to 5.2x10⁶/µL, which is 2-3 times that of platelets sequestered when processing only one cycle of whole anticoagulated blood. Once the plasma reduced platelet concentrate is extracted from the bowl 12 and collected within container 22, the system 10 may then return any blood components remaining within the bowl 12 and any plasma still remaining within the plasma container 18, step 416 of Fig. 4.

It should be noted that the surge elutriation technique may use a variety of fluids other than plasma to extract the platelets and/or the reduced plasma platelet product from the separation chamber (e.g., saline solution may be used). Additionally, the platelets that are reintroduced into the separation chamber may be re-anticoagulated to prevent the platelets from coagulating and/or clumping. For example, the platelet collection bag 20 or the reduced plasma platelet product bag 22 may be pre-loaded with a quantity of anticoagulant so that the platelets and/or reduced plasma platelet product mix with the anticoagulant as they are drawn from the separation chamber. Additionally or alternatively, sufficient anticoagulant may be added as the whole blood is withdrawn from the subject such that enough anticoagulant is still present in the platelets prior to reprocessing. In either scenario, the amount of anticoagulant added the whole blood and/or extracted platelets must be weighed against the safety of the subject. In particular, the amount of anticoagulant should be limited so as to prevent a large quantity of anticoagulant being returned to the subject.

It should also be noted that once the platelets and the reduced plasma platelet product are collected, a platelet preservative solution may be added to help preserve and store the platelets for later use. The preservative solution can be added to the platelets and platelet product after collection (e.g., from a separate bag or storage container 96), or the platelet collection bag 20 and the reduced plasma platelet product bag 22 may be pre-loaded with the additive solution.

By anticipating the total amount of plasma that will be returned to the donor and returning the plasma in multiple steps, various embodiments of the present invention are able to provide numerous advantages over the prior art. For example, by not returning all of the plasma at the end of the procedure, various embodiments of the present invention are able to greatly reduce patient discomfort and the risks associated with returning large volumes of citrated plasma. Additionally, by returning plasma during "dead" times, some embodiments of the present invention are able to reduce the overall procedure time, and the ECV/IVD at any given time during the procedure. Furthermore, because plasma may be returned during processing and the EVC/IVD is significantly reduced, various embodiments of the present invention do not need to return saline (or other compensation fluid) to the patient to reduce the ECV.

Although the embodiments described above discuss returning the plasma to the patient/donor, not all of the plasma needs to be returned. For example, some embodiments of the present invention may save a portion of the collected plasma. In such embodiments, the plasma may be filtered using F3 and stored within filtered plasma container 97.

If additional reduced plasma platelet product is required, each of the steps described above may now be repeated until a desired quantity of plasma reduced platelet product is collected. In various embodiments, plasma may be added to the plasma reduced platelet product so as to adjust the plasma reduced product to a predetermined volume or concentration.

Although the above embodiments are described as withdrawing whole blood from a patient/donor and returning unwanted components back to the donor, other embodiments of the present invention may be used for donor-free processing. For example, as discussed above, the venous access device 24 may be replaced with a whole blood bag (not shown) in cases in which the whole blood is to be first pooled and then supplied to the separation device 11. In such embodiments, the plasma from the plasma container 18 and the blood components remaining in the separation device 11 may be returned to one or more storage bags/containers (e.g., all into one container or into separate containers) or returned to the whole blood bag. Such embodiments would still have advantages over the prior art systems because of the greatly reduced processing time (e.g., because plasma is sent to the containers during dead times, etc.).

## Claims

1. A system for collecting plasma reduced platelets and anticipating plasma return comprising:
means for drawing a first volume of whole blood (24, 28) from a subject and returning blood components to the subject;
a blood component separation means (11, 12) for separating the drawn blood into a first blood component and a second blood component, the blood component separation means (11, 12) configured to send the first blood component to a first blood component container (18) and the second blood component to a second blood component container (20);
a first return means (41) fluidly connecting the means for drawing a first volume of whole blood (24, 28) and the blood component separation means (11, 12), the first return means (41) configured to return blood components remaining in the separation means (11, 12) to the subject;
a recirculation means (40, 43) connecting the second blood component container (20) and the separation means (11, 12), wherein the recirculation means (40, 43) is configured to reintroduce the second blood component within the second blood component container (20) into the separation means (11, 12); and
a second return means (93) fluidly connecting the first blood component container (18) and the first return means (41) and configured to return the first blood component within the first blood component container (18) to the subject;
**characterized in that** the second return means (93) is configured to bypass the blood component separation means (11, 12) when returning the first blood component within the first blood component container (18) to the subject.

2. A system according to claim 1, wherein the means for drawing whole blood (24, 28) includes a first pump (PI), the first pump (P1) configured to return the first blood component within the first blood component container (18) to the subject.

3. A system according to claim 2, wherein the first pump (P1) is also configured to return the blood components remaining in the separation means (11, 12) to the subject.

4. A system according to claim 1, further comprising a controller for controlling the flow of fluids through the system, the controller configured to repeatedly draw whole blood from the donor into the separation means (11, 12), extract first and second blood components from the separation means (11, 12), return first blood components within the first blood component container (18) to the subject using the second return means (93), and return remaining components in the separation means (11, 12) back to the subject using the first return means (41), wherein after a predetermined volume of second blood component has been sequestered in the second blood component container (20), the second blood component from the second blood component container (20) is reintroduced into the separation means (11, 12).

5. A system according to claim 4, wherein the controller is configured to calculate at least one of an extra-corporeal volume and an intra-vascular deficit, the amount of first blood component returned using the second return means (93) being based at least in part on the at least one of an extra-corporeal volume and an intra-vascular deficit.

6. A system according to claim 1, wherein the second blood component is platelets.

7. A system according to claim 1, wherein the separation means (11, 12) is configured to separate the whole blood into a third blood component in addition to the first and second blood components.

8. A system according to claim 7, wherein blood components remaining in the separation means (11, 12) include the third blood component.

9. A system according to claim 7, wherein the third blood component is red blood cells.

10. A system according to claim 1, wherein the system is configured to remove the second blood component from the separation device (11, 12) using a surge elutriation method.

11. A system according to claim 10, wherein the surge elutriation method includes reintroducing the first blood component into the blood component separation means (11, 12) through the recirculation means (40, 42) at an increasing rate until the second blood component is removed from the blood component separation means (11, 12).

12. A system according to claim 1, further comprising means for introducing anticoagulant (32) into the drawn whole blood.

13. A system according to claim 1, wherein the second blood component is platelets, the system configured to reintroduce the second blood component into the blood component separation means (11, 12) after a predetermined amount of platelets is collected within the second blood component container (20) so as to extract a plasma reduced platelet product from the separation means (11, 12).

14. A system according to claim 1, further comprising a reintroduction means (43) fluidly connecting the second blood component container (20) and the blood component separation means (11, 12), wherein the system is configured to reintroduce the second blood component within the second blood component container (20) into the blood component separation means (11, 12) when a second volume of whole blood is withdrawn from the subject, thereby creating an enlarged layer of the second blood component within the blood component separation means (11, 12), the system also configured to remove the enlarged layer of second blood component from the blood component separation means (11, 12) using a surge elutriation method.

## Patentansprüche

1. System zum Sammeln von plasmaverminderten Blutplättchen und Vorwegnahme von Plasmarückführung, das Folgendes umfasst:
ein Mittel zum Entnehmen eines ersten Volumens an Vollblut (24, 28) von einem Subjekt und Zurückführen von Blutbestandteilen zu dem Subjekt,
ein Blutbestandteil-Trennungsmittel (11, 12) zum Trennen des entnommenen Blutes in einen ersten Blutbestandteil und einen zweiten Blutbestandteil, wobei das Blutbestandteil-Trennungsmittel (11, 12) konfiguriert ist, um den ersten Blutbestandteil zu einem ersten Blutbestandteilbehälter (18) zu schicken und den zweiten Blutbestandteil zu einem zweiten Blutbestandteilbehälter (20) zu schicken,
ein erstes Rückführungsmittel (41), welches das Mittel zum Entnehmen eines ersten Volumens an Vollblut (24, 28) und das Blutbestandteil-Trennungsmittel (11, 12) fluidmäßig verbindet, wobei das erste Rückführungsmittel (41) konfiguriert ist, um Blutbestandteile, die in dem Trennungsmittel (11, 12) verbleiben, zu dem Subjekt zurückzuführen,
ein Rücklaufmittel (40, 43), das den zweiten Blutbestandteilbehälter (20) und das Trennungsmittel (11, 12) verbindet, wobei das Rücklaufmittel (40, 43) konfiguriert ist, um den zweiten Blutbestandteil innerhalb des zweiten Blutbestandteilbehälters (20) wieder in das Trennungsmittel (11, 122) einzuleiten, und
ein zweites Rückführungsmittel (93), welches den ersten Blutbestandteilbehälter (18) und das erste Rückführungsmittel (41) fluidmäßig verbindet und konfiguriert ist, um den ersten Blutbestandteil innerhalb des ersten Blutbestandteilbehälters (18) zu dem Subjekt zurückzuführen,
**dadurch gekennzeichnet, dass** das zweite Rückführungsmittel (93) konfiguriert ist, um das Blutbestandteil-Trennmittel (11, 12) zu umgehen, wenn der erste Blutbestandteil innerhalb des ersten Blutbestandteilbehälters (18) zu dem Subjekt zurückgeführt wird.

2. System nach Anspruch 1, wobei das Mittel zum Entnehmen von Vollblut (24, 28) eine erste Pumpe (P1) einschließt, wobei die erste Pumpe (P1) konfiguriert ist, um den ersten Blutbestandteil innerhalb des ersten Blutbestandteilbehälters (18) zu dem Subjekt zurückzuführen.

3. System nach Anspruch 2, wobei die erste Pumpe (P1) ebenfalls konfiguriert ist, um Blutbestandteile, die in dem Trennungsmittel (11, 12) verbleiben, zu dem Subjekt zurückzuführen.

4. System nach Anspruch 1, das ferner eine Steuerung zum Steuern des Stroms von Fluids durch das System umfasst, wobei die Steuerung konfiguriert ist, um wiederholt Vollblut von dem Spender in das Trennungsmittel (11, 12) zu entnehmen, um einen ersten und einen zweiten Blutbestandteil aus dem Trennungsmittel (11, 12) zu extrahieren, um erste Blutbestandteile innerhalb des ersten Blutbestandteilbehälters (18) unter Verwendung des zweiten Rückführungsmittels (93) zu dem Subjekt zurückzuführen und um verbleibende Bestandteile in dem Trennungsmittel (11, 12) unter Verwendung des ersten Rückführungsmittels (41) wieder zu dem Subjekt zurückzuführen, wobei, nachdem ein vorbestimmtes Volumen des zweiten Blutbestandteils in dem zweiten Blutbestandteilbehälter (20) abgesondert worden ist, der zweite Blutbestandteil aus dem zweiten Blutbestandteilbehälter (20) wieder in das Trennungsmittel (11, 12) eingeleitet wird.

5. System nach Anspruch 4, wobei die Steuerung konfiguriert ist, um mindestens eines von einem extrakorporalen Volumen und einem intravaskulären Defizit zu berechnen, wobei die Menge des ersten Blutbestandteils, die unter Verwendung des zweiten Rückführungsmittels (93) zurückgeführt wird, mindestens zum Teil auf dem mindestens einen von einem extrakorporalen Volumen und einem intravaskulären Defizit beruht.

6. System nach Anspruch 1, wobei der zweite Blutbestandteil Blutplättchen sind.

7. System nach Anspruch 1, wobei das Trennungsmittel (11, 12) konfiguriert ist, um das Vollblut zusätzlich zu dem ersten und dem zweiten Blutbestandteil in einem dritten Blutbestandteil zu trennen.

8. System nach Anspruch 7, wobei die Blutbestandteile, die in dem Trennungsmittel (11, 12) verbleiben, den dritten Blutbestandteil einschließen.

9. System nach Anspruch 7, wobei der dritte Blutbestandteil rote Blutkörperchen sind.

10. System nach Anspruch 1, wobei das System konfiguriert ist, um den zweiten Blutbestandteil unter Verwendung eines Schwallschlämmverfahrens aus dem Trennungsmittel (11, 12) zu entfernen.

11. System nach Anspruch 10, wobei das Schwallschlämmverfahren das Wiedereinleiten des ersten Blutbestandteils in das Blutbestandteil-Trennungsmittel (11, 12) durch das Rücklaufmittel (40, 42) mit einer zunehmenden Rate, bis der zweite Blutbestandteil aus dem Blutbestandteil-Trennungsmittel (11, 12) entfernt ist, einschließt.

12. System nach Anspruch 1, das ferner Mittel zum Einleiten von einem Gerinnungshemmer (32) in das entnommene Vollblut umfasst.

13. System nach Anspruch 1, wobei der zweite Blutbestandteil Blutplättchen sind, wobei das System konfiguriert ist, um den zweiten Blutbestandteil wieder in das Blutbestandteil-Trennungsmittel (11, 12) einzuleiten, nachdem eine vorbestimmte Menge an Blutplättchen innerhalb des zweiten Blutbestandteilbehälters (20) gesammelt ist, um so ein plasmavermindertes Blutplättchenerzeugnis aus dem Trennungsmittel (11, 12) zu extrahieren.

14. System nach Anspruch 1, das ferner ein Wiedereinleitungsmittel (43) umfasst, das den zweiten Blutbestandteilbehälter (20) und das Blutbestandteil-Trennungsmittel (11, 12) fluidmäßig verbindet, wobei das System konfiguriert ist, um den zweiten Blutbestandteil innerhalb des zweiten Blutbestandteilbehälters (20) wieder in das Blutbestandteil-Trennungsmittel (11, 12) einzuleiten, wenn ein zweites Volumen an Vollblut von dem Subjekt entnommen wird, wodurch eine vergrößerte Schicht des zweiten Blutbestandteils innerhalb des Blutbestandteil-Trennungsmittels (11, 12) erzeugt wird, wobei das System ebenfalls konfiguriert ist, um die vergrößerte Schicht des zweiten Blutbestandteils unter Verwendung eines Schwallschlämmverfahrens aus dem Blutbestandteil-Trennungsmittel (11, 12) zu entfernen.

## Revendications

1. Système de collecte de plaquettes à plasma réduit et d'anticipation de la réinjection du plasma, comprenant :
un moyen pour prélever un premier volume de sang total (24, 28) d'un sujet et pour réinjecter des composants sanguins au sujet ;
un moyen de séparation du composant sanguin (11, 12) pour séparer le sang total dans un premier composant sanguin et un deuxième composant sanguin, le moyen de séparation du composant sanguin (11, 12) étant configuré pour transférer le premier composant sanguin vers un premier récipient de composant sanguin (18) et le deuxième composant sanguin vers un deuxième récipient de composant sanguin (20) ;
un premier moyen de réinjection (41) connectant fluidiquement le moyen destiné à prélever un premier volume de sang total (24, 28) et le moyen de séparation du composant sanguin (11, 12), le premier moyen de réinjection (41) état configuré pour réinjecter les composants sanguins restant dans le moyen de séparation (11, 12) au sujet ;
un moyen de recirculation (40, 43) connectant le deuxième récipient de composant sanguin (20) et le moyen de séparation (11, 12), dans lequel le moyen de recirculation (40, 43) est configuré pour réintroduire le deuxième composant sanguin contenu dans le deuxième récipient de composant sanguin (20) dans le moyen de séparation (11, 12) ; et
un deuxième moyen de réinjection (93) connectant fluidiquement le premier récipient de composant sanguin (18) et le deuxième moyen de réinjection (41) et configuré pur réinjecter le premier composant sanguin contenu dans le premier récipient de composant sanguin (18) au sujet ;
**caractérisé en ce que** le deuxième moyen de réinjection (93) est configuré pour contourner le moyen de séparation du composant sanguin (11, 12) lors de la réinjection du premier composant sanguin contenu dans le premier récipient de composant sanguin (18) au sujet.

2. Système selon la revendication 1, dans lequel le moyen destiné à prélever le sang total (24, 28) inclut une première pompe (P1), la première pompe (P1) étant configurée pour réinjecter le premier composant sanguin contenu dans le premier récipient de composant sanguin (18) au sujet.

3. Système selon la revendication 2, dans lequel la première pompe (P1) est également configurée pour réinjecter les composants sanguins restant dans le moyen de séparation (11, 12) au sujet.

4. Système selon la revendication 1, comprenant en outre un moyen de commande pour contrôler l'écoulement de fluides à travers le système, le moyen de commande étant configuré pour prélever de manière répétée le sang total du donneur dans le moyen de séparation (11, 12), pour extraire les premier et deuxième composants sanguins du moyen de séparation (11, 12), pour réinjecter les premiers composants sanguins contenus dans le premier récipient de composant sanguin (18) au sujet en utilisant le deuxième moyen de réinjection (93) et pour réinjecter les composants restant dans le moyen de séparation (11, 12) au sujet en utilisant le premier moyen de réinjection (41), dans lequel, après la séquestration d'un volume prédéterminé du deuxième composant sanguin dans le deuxième récipient de composant sanguin (20), le deuxième composant sanguin provenant du deuxième récipient de composant sanguin (20) est réintroduit dans le moyen de séparation (11, 12).

5. Système selon la revendication 4, dans lequel le moyen de commande est configuré pour calculer au moins un d'un volume extracorporel ou d'un déficit intravasculaire, la quantité du premier composant sanguin réinjecté par l'intermédiaire du deuxième moyen de réinjection (93) étant basée au moins en partie sur au moins un du volume extracorporel ou du déficit intravasculaire.

6. Système selon la revendication 1, dans lequel le deuxième composant sanguin sont des plaquettes.

7. Système selon la revendication 1, dans lequel le moyen de séparation (11, 12) est configuré pour séparer le sang total en un troisième composant sanguin, en plus des premier et deuxième composants sanguins.

8. Système selon la revendication 7, dans lequel les composants sanguins restant dans le moyen de séparation (11, 12) incluent le troisième composant sanguin.

9. Système selon la revendication 7, dans lequel le troisième composant sanguin sont des globules rouges.

10. Système selon la revendication 1, dans lequel le système est configuré pour retirer le deuxième composant sanguin du dispositif de séparation (11, 12) en appliquant un procédé d'élutriation par surpression.

11. Système selon la revendication 10, dans lequel le procédé d'élutriation par surpression inclut la réintroduction du premier composant sanguin dans le moyen de séparation du composant sanguin (11, 12) par l'intermédiaire du moyen de recirculation (40, 42) à une vitesse accrue jusqu'au retrait du deuxième composant sanguin du moyen de séparation du composant sanguin (11, 12).

12. Système selon la revendication 1, comprenant en outre un moyen pour introduire un anticoagulant (32) dans le sang total prélevé.

13. Système selon la revendication 1, dans lequel le deuxième composant sanguin sont des plaquettes, le système étant configuré pour réintroduire le deuxième composant sanguin dans le moyen de séparation du composant sanguin (11, 12) après la collecte d'une quantité prédéterminée de plaquettes dans le deuxième récipient de composant sanguin (20) pour extraire un produit de plaquette à plasma réduit du moyen de séparation (11, 12).

14. Système selon la revendication 1, comprenant en outre un moyen de réintroduction (43) connectant fluidiquement le deuxième récipient de composant sanguin (20) et le moyen de séparation du composant sanguin (11, 2), dans lequel le système est configuré pour réintroduire le deuxième composant sanguin contenu dans le deuxième récipient du composant sanguin (20) dans le moyen de séparation du composant sanguin (11, 12) lorsqu'un deuxième volume de sang total est prélevé du sujet, formant ainsi une couche élargie du deuxième composant sanguin dans le moyen de séparation du composant sanguin (11, 12), le système étant également configuré pour retirer la couche élargie du deuxième composant sanguin du moyen de séparation du composant sanguin (11, 12) en appliquant un procédé d'élutriation par surpression.
